# EUROPEAN PATENT SPECIFICATION

(11) **EP 2 537 510 B1**
(45) Date of publication and mention of the grant of the patent: **01.03.2017**
(21) Application number: 11171397.0
(22) Date of filing: 24.06.2011
(51) Int. Cl.: A61K 8/02, A61K 8/04, A61K 8/44, A61K 8/73, A61K 8/81, A61Q 19/10, A61Q 19/00

(54) **Personal cleaning composition comprising gelling and foaming agents for tissue impregnation**
Körperreinigungszusammensetzung enthaltend Gelier- und Schaummittel zur Tücherimpregnierung
Composition de nettoyage personnel comprenant des agents gélifiant et moussant pour l'imprégnation de papier

(43) Date of publication of application: 26.12.2012
(73) Proprietor: SCA TISSUE FRANCE, 93400 ST-OUEN (FR)
(72) Inventor: Annaheim, Stéphanie, 68520 Burnhaupt-Le-Haut (FR); Bret, Bruno, 68920 Wintzenheim (FR); Walgenwitz, Hervé, 68170 Rixheim (FR); Ruppel, Rémy, 68320 Durrenentzen (FR); Clermont, Anne-Gaëlle, 68000 Colmar (FR); Probst, Pierre, 68770 Ammerschwihr (FR); Jeannot, Sébastien, 68320 Holtzwihr (FR)
(74) Representative: Gevers & Orès

(56) References cited:
- EP-A1- 1 982 689
- DE-A1- 10 221 813
- US-A- 3 541 581
- US-A1- 2003 228 337
- US-A1- 2008 108 534
- US-A1- 2010 297 191

## Description

The present invention relates to a composition for cleaning, in particular for cleansing of the skin and/or mucous membranes, in particular a personal care cleansing composition, to be applied onto tissue paper, in particular toilet paper.

The present invention also relates to the use of said composition.

Improving personal care hygiene, when using the bathroom, is a constant need. Regular and effective cleaning not only improves the level of cleanliness but may also prevent irritation and/or, in some cases, prevent occurrence of infections and/or promote healing of disorders. Thorough cleanliness of the desired surface can be obtained by the conventional water/soap/drying routine. However, when travelling or working away from home, such method is not very convenient.

An alternative is the use of wet wipes or wet folded toilet paper under the faucet. However, these methods are not necessarily the most effective: plain water tears the toilet paper often making it unusable and wet wipes must be kept moist and hence may require specific packaging. Moreover, the waste created with soiled wet wipes, cannot be flushed like toilet paper as they do not disintegrate rapidly in water and can clog the pipes.

It is thus highly desirable to improve personal care hygiene when using bathroom, by ensuring thorough cleanliness of the desired body surface by eliminating the need of the above-mentioned methods.

Foamable cleansing compositions are good alternatives to previously mentioned methods. Such compositions have already been described in DE 10 2009 013 268. A foamable cleansing composition, Aaah^{®} commercialized by Aaah Company, is currently on the market. This composition essentially contains water, a foamable surfactant, an emollient, a soothing agent, a deodorant and natural extracts. Despite their improvements over the previously mentioned methods, these foamable compositions have some drawbacks. The main drawback of these compositions is the insufficient stability of the foam over time once applied onto the tissue paper, causing a too quick absorption of the foam by the paper, and hence a too quick "soak through" and tear of the paper by the foam.

US 2010/0297191 which may be considered as a possible closest state of the art, discloses cleansing and/or care compositions that are shear-thinning and thixotropic. The compositions of US 2010/0297191 are not foamable compositions.

EP 1 982 689 which may be considered as a possible closest state of the art is directed to an anti-microbial wet wipe comprising a substrate and a lotion whose pH is between 2.5 and 5.4. In Table 1, EP 1 982 689 describes a lotion containing xanthan gum as a viscosity modifier, and non-ionic surfactants Cremophor CO 40 and Abil Care 85.

The term "soak through paper properties" refers to the ability of the composition to soak, penetrate, diffuse and pass/go through the sheets/layers of the tissue paper piled on each other. The faster a composition soaks, penetrates, diffuses and passes/goes through the sheets of tissue paper, the faster it can tear said sheets, making them unusable.

Therefore, a need currently exists for a composition for cleansing of the skin and/or mucous membranes, which:
- has improved soak through paper properties (does not soak through the paper),
- has an effective cleansing action,
- does not need to be rinsed, and
- leaves a clean, soothing and pleasant feel.

More particularly, a need exists for a composition for cleansing of the skin and/or mucous membranes, which, once applied onto the tissue paper, more particularly toilet paper, is capable of remaining on the surface of the paper in the form of a foam and not soak through the paper during use, thereby allowing efficient cleansing.

In addition, a need exists for a cleansing composition as indicated above that is environmentally-friendly, without extra waste, easy to handle and that does not require costly ingredients.

Furthermore, a need exists for composition for cleansing of the skin and/or mucous membranes, that is capable of producing high quality foam (high stability over time and good soak through paper properties), with a very good cleansing action, good skin tolerance and skin care action, and without needing to be rinsed.

The present invention addresses these and other needs in the art by providing a composition according to claim 1.

In a first embodiment described but not part of the invention, a composition comprising:
- at least 70% by weight of water, relative to the total weight of the composition;
- 0.1 to 1 % by weight of at least one gelling agent, relative to the total weight of the composition, and
- 0.1 to 10% by weight, of at least one surfactant including at least one foaming surfactant, relative to the total weight of the composition.

Preferably the composition according to the invention, satisfies at least one of the following conditions:
- once transformed into foam and applied onto the surface of several sheets of tissue paper placed one on top of each other, the time required for said composition to soak through said sheets of paper, with or without applied pressure, is at least two times greater than the time required for a composition without a gelling agent (i.e. comparative examples 1 and 2) to soak through said sheets of paper, and/or
- the foam stability of said composition is such that after two minutes, the said composition is transformed into liquid at least two times less faster than a composition without a gelling agent (i.e. comparative examples 1 and 2), and/or
- once transformed into foam and applied onto the surface of several sheets of tissue paper, the time required to tear said sheets of paper under a mechanical stress, is at least two times less than the time required to tear said sheets of paper onto which a composition without a gelling agent (i.e. comparative examples 1 and 2) is applied.

In an alternative embodiment described but not part of the invention, a composition comprising water, at least one gelling agent and at least one surfactant including at least one foaming surfactant, wherein said composition satisfies at least one of the following conditions:
- once transformed into foam and applied onto the surface of several sheets of tissue paper placed one on top of each other, the time required for said composition to soak through said sheets of paper, with or without applied pressure, is at least two times greater than the time required for a composition without a gelling agent (i.e. comparative examples 1 and 2) to soak through said sheets of paper, and/or
- the foam stability of said composition is such that after two minutes, the said composition is transformed into liquid at least two times less faster than a composition without a gelling agent (i.e. comparative examples 1 and 2), and/or
- once transformed into foam and applied onto the surface of several sheets of tissue paper, the time required to tear said sheets of tissue paper under a mechanical stress, is at least two times less than the time required to tear said sheets of paper onto which a composition without a gelling agent (i.e. comparative examples 1 and 2) is applied.

In the alternative embodiment described but not part of the invention, the composition comprises:
- at least 70 % by weight of water, relative to the total weight of the composition, and/or
- 0.1 to 1 % by weight of at least one gelling agent, relative to the total weight of the composition, and/or
- 0.1 to 10 % by weight of at least one surfactant including at least one foaming surfactant, relative to the total weight of the composition.

All features and aspects disclosed in greater detail hereafter, apply to both of the aforementioned embodiments of the present invention.

The above-mentioned tissue paper is preferably a 1-ply CWP (conventional wet-pressed) + 1-ply TAD (through-air-dried) toilet paper weighing 38.5 g/m².

Once the composition according to the present invention is transformed into foam and applied onto the surface of 2 sheets of tissue paper placed one on top of each other, preferably 2 sheets of a 1-ply CWP + 1-ply TAD toilet paper weighing 38.5 g/m², the time required for said composition to soak through said sheets of paper without applied pressure, is at least two times greater than the time required for a composition without a gelling agent (i.e. comparative examples 1 and 2) to soak through said sheets of paper.

Once the composition according to the present invention is transformed into foam and applied onto the surface of 4 sheets of tissue paper placed one on top of each other, preferably 4 sheets of a 1-ply CWP + 1-ply TAD toilet paper weighing 38.5 g/m², the time required for said composition to soak through said sheets of paper with applied pressure, is at least two times greater than the time required for a composition without a gelling agent (i.e. comparative examples 1 and 2) to soak through said sheets of paper.

Once the composition according to the present invention is transformed into foam and applied onto the surface of 3 sheets of tissue paper placed one on top of each other, preferably 3 sheets of a 1-ply CWP + 1-ply TAD toilet paper weighing 38.5 g/m², the time required to tear said sheets of paper under mechanical stress, is at least two times less than the time required to tear said sheets of paper onto which a composition without a gelling agent (i.e. comparative examples 1 and 2) is applied.

The cleansing composition of the invention is for topical application, meaning an external application to the skin and/or mucous membranes. Since the composition is for topical application, it comprises a physiologically acceptable medium. The term "physiologically acceptable medium" is intended to mean a medium compatible with the skin and/or mucous membranes.

The composition according to the invention is foamable, meaning that when combined with a gas, preferably air, it is capable of being transformed into a foam.

The composition of the invention brings effective cleanliness with small amounts of surfactant(s) and leaves a fresh and pleasant feel to the skin and improves the softness of the tissue paper on which it is applied. Furthermore, no rinsing is required with the composition of the invention.

The composition described but not part of the invention comprises at least 70% by weight, preferably 80% by weight of water, relative to the total weight of the composition. The amount of water can range, for example, from 70% to 95% by weight, preferably from 80% to 95% by weight. The composition of the present invention comprises more preferably from 85% to 95% by weight, relative to the total weight of the composition, including the stated values and all weights and weight ranges between stated values.

The composition may optionally contain, in addition to water, one or more solvents chosen from alcohols containing from 1 to 6 carbon atoms such as ethanol; polyols such as glycerol; glycols such as butylene glycol, sorbitol, isoprene glycol, propylene glycol, polyethylene glycols such as PEG-8. The amount of solvents used in the composition, in addition to water, can range from 0.5 to 20% by weight and preferably from 1 to 10% by weight, relative to the total weight of the composition, including the stated values and all weights and weight ranges between stated values.

The term gelling agent, also known as thickening agent, is intended to mean a polymer which swells in water and increases the viscosity of the composition, gives a better feeling, decreases the foaming properties of the composition, etc. The stability of the composition before and after being transformed into foam, is also improved by the presence of the gelling agent.

In the composition described but not part of the invention, the gelling agent can in particular be chosen from:
- acrylic polymers chosen from
   - acrylic and/or methacrylic acid homo- or co-polymers, their salts and esters such as sodium acrylate copolymer,
   - acrylic acid and acrylamide copolymers such as sodium polymethacrylate,
   - acrylates/C₁₀₋₃₀ alkyl acrylates copolymers or crosspolymers such as Pemulen^{®} polymers, polyacrylamidomethyl propane sulfonic acid and its derivatives (partially neutralized with ammonia and highly reticulated), Carbopol^{®} Ultrez 20 and Carbopol^{®} Ultrez 21;
   - carbomers such as Carbopols^{®} and more specifically Carbopols^{®} Ultrez 10;
   - vinyl polymers chosen from polyvinylpyrrolidone (PVP), methylvinyl ether and maleic anhydride copolymers, vinylacetate and crotonic acid copolymers, vinylpyrrolidone and caprolactam copolymers, or polyvinyl alcohols;
   - polyethylene glycols chosen from macrogols, carbowax, monowax, hydrocire, or lutrol;
   - polyquaterniums such as polyquaternium 10;
   - polysaccharides chosen from starches such as starch from cereal grains, xanthan gum, carrageenans, agar-agar, gelose, or alginates; resins and gums chosen from Arabic gum, guar gum, tara gum, locust bean gum, or karaya gum;
   - cellulose derivatives selected from hydroxypropylcellulose (HPC), hydroxyethylcellulose (HEC) such as different types of Natrosol^{®}, ethylhydroxyethyl cellulose, methylcellulose, hydroxypropyl methylcellulose, carboxymethyl cellulose, or quaternized derivatives of cellulose;
   - anionic, cationic, amphoteric or non-ionic chitin or chitosan polymers;
   - silica chosen from hydrophilic, fumed or silylated silica, such as Aerosils^{®} including Aerosil^{®}130, Aerosil^{®} R812;
   - clays and silicates chosen from montmorillonites, hectorites, bentonites, beidellites, saponites, vermiculites, stevensites or chlorites;
and mixtures thereof.

Clays such as montmorillonites, hectorites, bentonites, beidellites and saponites, are modified by chemical compounds selected from quaternary/tertiary amines, aminoacetates, imidazolines, amine soaps, fatty sulfates, alkyl aryl sulfonates, amine oxides, and mixtures thereof, in order to make them capable of swelling in oily media.

In a preferred embodiment described but not part of the invention, the gelling agent is chosen from:
- acrylic polymers chosen from acrylic and/or methacrylic acid homo- or co-polymers, their salts and esters, acrylic acid and acrylamide copolymers, acrylates/C₁₀₋₃₀ alkyl acrylates copolymers, polyacrylamidomethyl propane sulfonic acid and its derivatives, carbomers, preferably carbomers,
- cellulose derivatives selected from hydroxypropylcellulose, hydroxyethylcellulose, ethylhydroxyethyl cellulose, methylcellulose, hydroxypropyl methylcellulose, carboxymethyl cellulose, or quaternized derivatives of cellulose, preferably hydroxythylcellulose (HEC);
and mixtures thereof.

In the composition of the present invention, the gelling agent(s) is (are) chosen from carbomers, hydroxythylcellulose (HEC), and mixtures thereof.

When a composition is foamable, it is usually difficult to thicken the foaming medium while at the same time conserving the required properties, i.e. ability to foam in particular with a foam dispenser; good foam stability and reduced "soak through paper" properties when applied on tissue paper, in particular toilet paper. Hence, the choice of the gelling agent and the amount used in the composition of the invention is an important parameter. A cleansing composition comprising less than 0.1% by weight of gelling agent shows a too low "soak through properties" and foam stability. A cleansing composition comprising more than 1% by weight of gelling agent shows a too high viscosity and thus difficulty in transforming the composition into foam, in particular with a foam dispenser.

In a preferred embodiment described but not part of the invention, the total amount of gelling agent(s) in the composition is from 0.2 to 0.5 % by weight. In the composition of the present invention the total amount of gelling agent(s) in the composition is from 0.25 to 0.45%, by weight, relative to the total weight of the composition, including the stated values and all weights and weight ranges between stated values. The "total amount" is intended to mean the amount of all gelling agents(s) present in the composition.

As already indicated, the composition according to the invention comprises at least one surfactant including at least one foaming surfactant. This means that the composition may either contain only one or more foaming surfactant(s), or one or more other type(s) of surfactant(s) in addition to the foaming surfactant(s).

The term "foaming surfactant" is intended to mean a surfactant which has foaming properties when it is introduced in water.

The foaming surfactant(s) described but not part of the invention may be chosen from, but not limited to, the following surfactants:
- amphoteric surfactants chosen from
   - betaine derivatives chosen from alkylbetaines such as cocobetaine, alkylaminobetaines (alkyl amidobetaines) such as cocamidopropylbetaine,
   - sultaines such as hydroxysultaine,
   - alkyl polyaminocarboxylates such as cocoyl polyaminocarboxylate,
   - alkyl amphoacetates such as disodium cocamphodiacetate, or
   - imidazole derivatives;
- non-ionic surfactants chosen from
   - polycondensates of oxyethylene/oxypropylene chosen from glycerol polyoxyethylene and/or polyoxypropylene ether, fatty alcohol oxyethylene and/or oxypropylene ether such as ceteareths and more specifically ceteareth-30, fatty acid esters of glycerol oxyethylene and/or oxypropylene such as PEG-200 glyceryl monostearate, fatty acid esters of sorbitol polyoxyethylene and/or polyoxypropylene such as polysorbates and more specifically polysorbate-60, oxyethylene and oxypropylene copolymers, ethylene and propylene oxide condensates with fatty alcohols, polyethylene glycol fatty acid esters such as PEG-50 stearate, alkylpolyglucoside optionally oxyalkylated, alkylglucoside esters such as decyl glucoside, fatty acid esters of sorbitan optionally oxyethylated, polyoxyalkylated fatty acid esters,
   - amine derivatives chosen from N-alkylglucamine and N-acylmethylglucamine derivatives, amines, amine oxides, polyethoxylated or polyglycerolated fatty amines, polyglycerolated diglycolamides,
   - silicone derivatives chosen from dimethicone copolyol, dimethicone copolyol benzoate, cyclomethicone/dimethicone copolyol,
   - saccharide derivatives chosen from esters and ethers of monosaccharide such as sucrose stearate, fatty acid esters of saccharose such as sucrose stearate,
   - ethylene oxide derivatives chosen from alkoxylated sugar esters such as PEG-120 methylglucose dioleate;
and mixtures thereof.

The composition of the invention comprises at least one foaming surfactant, said foaming surfactant(s) being a betaine derivative chosen from alkylbetaines such as cocobetaine, alkyl amidobetaines such as cocamidopropylbetaine, and mixtures thereof.

The total weight of foaming surfactant(s) in the composition ranges from 0.1 to 10 % by weight, preferably from 0.5 to 6% by weight, relative to the total weight of the composition, including the stated values and all weights and weight ranges between stated values. The "total weight" in this embodiment is intended to mean the weight of all foaming surfactants present in the composition.

As previously mentioned, the composition according to the invention may only contain foaming surfactant(s). The composition may further contain or one or more other surfactant(s) in addition to the foaming surfactant(s). The total weight of surfactant(s) including the foaming surfactant(s) in the composition, ranges from 0.1 to 10% by weight, preferably from 0.5 to 6% by weight, relative to the total weight of the composition, including the stated values and all weights and weight ranges between stated values. The "total weight" is intended to mean the weight of all surfactants present in the composition.

The additional surfactants are mainly intended to lower the surface tension of water and other liquids or that between a liquid and a solid. They may thus act, for example, as detergents, wetting agents, emulsifiers and dispersants. They may also have foaming properties.

The additional surfactant(s) may be chosen from:
- anionic surfactants chosen from
   - anionic derivatives of proteins (apple, wheat, soya, oat, silk protein) and polypeptide derivatives;
   - carboxylates chosen from amido ether carboxylates such as sodium lauryl amido ether carboxylate, polyoxthyethylene carboxylates such as sodium lauryl ether carboxylate, fatty acid salts having an C₁₆₋₂₂ alkyl chain neutralized by an organic or inorganic base, which constitute soaps (alkaline soaps amine soaps of formula RCOOM with M being Na, K, NH₄⁺ or triethanolamine and R= C₁₆₋₂₂ alkyl chain);
   - aminoacid derivatives chosen from alkylsarcosinates such as sodium lauroyl sarcosinate (C₁₂H₂₅-CON(CH₃)-CH₂-COONa), alalinates such as sodium N-lauroyl N-methyl amido propionate, glutamates such as triethanolamine cocoyl glutamate, aspartates such as ethanolamine N-lauroyl aspartate or ethanolamine N-myristoyl aspartate, glycinates such as sodium N-cocyl glycinate, citrates such as citric monoester of coco alcohol ethoxylate;
   - taurates such as sodium salt of palm oil methyltaurate;
   - sulfate derivatives chosen from sodium, ammonium, potassium, triethanolamine alkyl sulfates such as sodium lauryl sulfate; sodium, ammonium, potassium, triethanolamine alkyl ether sulfates such as sodium lauryl ether sulfate;
   - sulfonate derivatives chosen from paraffins and olein solfonates such as sodium alpha olefin sulfonate, alkyl sulfoacetates (R-SO₃-CH₂-COOM), isethionates (R-COO-CH₂-CH₂-SO₃Na) such as sodium cocoylisethionate;
   - alkyl polyglucoside anionic derivatives chosen from sulfosuccinates, citrates, tartrates, carbonates and ethers of glycerol obtained from alkylglucosides such as mono-sulfosuccinate of lauric alcohol; or
   - phosphated derivatives chosen from sodium alkyl phosphates (RO-PO₃-Na₂) or alkyl ether phosphates de sodium R-(C₂H₄O)ₙPO₃Na₂;
- cationic surfactants chosen from quaternary ammoniums, alkylpyridinium chlorides, alkylammonium saccharinates, or aminoxydes having a pH < 6.5;
and mixtures thereof.

The composition of the invention may contain further additives and/or active agents chosen from, for example:
- preservatives or preserving agents, non limitative examples thereof being chlorhexidine, sodium chlorate; chlorphenesin; phenoxyethanol such as Phenoxetol^{®}; a mixture of phenoxyethanol, methylparaben and ethylparaben such as Phenonip^{®}; a mixture of phenoxyethanol, methylparaben and propylparaben such as Nipaguard BPX^{®}; PEG-4 laurate such as Nipaguard IPF^{®}; iodopropynyl butylcarbamate; a mixture of triethylene glycol, benzyl alcohol and propylene glycol such as Nipaguard CMB^{®}; sodium hydroxymethylglycinate such as Nipaguard SMG^{®}, sorbic acid, dehydroacetic acid, sodium benzoate, potassium sorb ate, lyaminopropyl biguanide (PAPB), benzoic acid;
- sequestering or chelating agents such as ethylenediaminetetraacetic acid (EDTA), ethylenediamine tetra(methylene phosphonic acid) (EDTMP), diethylenetriamine penta(methylene phosphonic acid) (DTPMP), nitrilotriacetic acid (NTA), pentetic acid or diethylene triamine pentaacetic acid (DTPA);
- antioxidants;
- fragrances such as eucalyptus oil, clove oil, jojoba oil, lavender oil; fleur d'eau MIP 1101016 from Expressions Parfumées;
- pH modifiers such as sodium hydroxide, citric acid;
- antiseptics; antimicrobials such as benzoyl peroxide, salicylic acid, triclosan;
- natural extracts having for example soothing, moisturizing, and healing properties, such as aloe vera;
- soothing agents or emollients such as allantoin; panthenol; polyols such as glycerol or glycerin, sorbitol; glycols such as butylene glycol, isoprene glycol; polyethylene glycols such as PEG-8, methoxypolyethylene glycols;
and mixtures thereof.

The amounts of these various additives are those conventionally used in this field, and complete the weight of the composition to a total weight of 100%. These additives and the concentrations thereof should be such that they do not modify the property desired for the composition of the invention and that they do not destabilize it.

In a preferred embodiment, the composition according to the invention contains at least a soothing agent or emollient. The soothing agents or emollients are preferably chosen from panthenol; polyols such as glycerol or glycerin; glycols such as butylene glycol, and mixtures thereof.

The amount of emollients in the composition ranges, for example, from 0.1 to 5% by weight relative to the total weight of the composition, including the stated values and all weights and weight ranges between stated values.

According to a particular embodiment of the invention, the composition comprises:
- 88 to 92 % by weight of water,
- 0.25 to 0.45 % by weight of a gelling such as carbomer and/or hydroxyethylcellulose,
- 1.5 to 2.5 % by weight of a foaming surfactant such as cocamidopropylbetaine,
- 0.2 to 2.5 % by weight of emollients such as butylene glycol and/or panthenol,
- 0.1 to 1 % by weight of preservatives such as caprylyl glycol and/or phenoxyethanol,
- 0.5 to 1.5 % by weight of a natural extract such as aloe vera,
- 0.15 to 2.7 % by weight of a pH modifier such as sodium hydroxide or citric acid,
- 0.1 to 0.15 % by weight of a chelating agent such as EDTA,
- 0.1 to 0.2 % by weight of a fragrance such as Fleur d'ea MIP 1101016,
all weights being relative to the total weight of the composition.

The composition according to the invention is preferably in the form of a gel.

The composition of the invention has a viscosity ranging, for example, from 0.001 to 1 Pa.s, measured at a temperature of 25°C, using a Brookfield LV DV I + from BROOKFIELD ENGINEERING LABORATORIES, INC. Preferably the viscosity of the composition according to the present invention ranges from 0.001 to 0.500 Pa.s, more preferably from 0.010 to 0.350 Pa.s. The device is equipped with a set of four spindles: LV1, LV2, LV3 and LV4. The following spindles (sp) and rotational speeds (v) are used:

| | |
|---|---|
| - sp 1 / v 100 rpm | for η < 0.1 Pa.s (100 cP) |
| - sp 2 / v 50 rpm | for 0.1 Pa.s (100 cP) < η < 1 Pa.s (1000 cP) |
| - sp 2 / v 10 rpm | for 1 Pa.s (1000 cP) < η < 3 Pa.s (3000 cP) |
| - sp 3/ v 50/20/10 rpm | for 3 Pa.s (3000 cP) < η < 6 Pa.s (6000 cP). |

The composition of the invention is foamable meaning that it is specifically formulated to form a foam when aerated. Said foam may be formed by using, for example, a foam dispenser.

Preferably, the foam dispenser is easy to use, forms foam instantly, in a precise dosage, allows an improved spread of effective ingredients, and without use of propellants.

The composition of the invention may be contained in a dispensing foam pump container using, for example, a positive displacement pump that acts directly on the composition. The pump draws the composition up a siphon tube from the bottom of the container, and the composition is forced out a nozzle.

For example, the dispensing foam pump container may be an aerosol container. In an aerosol container, the composition is maintained under pressure sufficient to cause foam formation when dispensed. Of particular advantage, however, the composition of the instant invention is foamable without necessity of being placed in an aerosol container.

Airspray pump foamers allowing precise mixing of the composition and air without using gas propellants, are preferred. Their use results in a high quality foam preferably with each single stroke.

Foam dispensers may be chosen from F2-L11, F3-L11, G3-L11, T1-L11, WRT4-L11, WRT-L11, F2-L9, F3-L9, G3-L9, T1-L9, WRT4-L9, WRT-L9, F2-L7, F3-L7, G3-L7, T1-L7, WRT4-L7, or WRT-L7 sold by Rexam. Other foam dispensers supplied by other suppliers may also be used.

Automatic touchless foam dispensers may also be used. An example of such automatic touchless foam dispenser is: enMotion^{®} Automated Touchless Soap Dispenser.

Said foamer dispensers entrain air in the composition as it is dispensed.

Another object of the present invention is the use of the composition according to the invention for cleansing of the skin and/or mucous membrane.

A further object of the invention is the use of the composition according to the invention with tissue paper, in particular toilet paper, for cleansing of the skin and/or mucous membrane.

A yet another object described is a method for cleansing skin and/or mucous membrane comprising:
(i) applying the composition according to the invention to a tissue paper, in particular toilet paper, and
(ii) cleansing the areas in need of being cleansed.
In step (i), when applied onto the tissue paper, the composition is in the form of a foam. The cleansing step (ii) may be carried out by wiping the areas to be cleansed with the tissue paper comprising said foamed composition on its surface.

The present description also relates to a method for cleansing skin and/or mucous membrane wherein the composition according to the invention is
(i) applied directly on the areas to be cleansed, and
(ii) cleansing said areas by wiping with a tissue paper, in particular toilet paper.

The cleaning composition according to the invention may be applied onto all types of tissue papers such as hankies, facial tissues, tissue wipes, hand towels, kitchen towels, for cleaning any type of supports, and in particular for cleansing the skin or the mucous membranes. In a preferred embodiment the tissue paper is particular toilet paper. In a still more preferred embodiment, the tissue paper, in particular toilet paper, does not contain a wet strength agent.

The composition may also be applied directly on the skin and/or mucous membrane to be cleansed, and then rinsed off with water. However, this is not a preferred embodiment of the present invention.

The present invention further concerns a kit for cleansing of the skin and/or mucous membranes comprising the composition according to the invention and a dispensing foam pump container. Said kit may further comprise tissue paper, in particular toilet paper.

The present invention also concerns a container provided with a dispensing foam pump and containing a composition according to the invention.

The present invention further concerns an automatic foam dispenser containing a composition according to the invention.

The present invention also concerns the use of a composition according to the invention with tissue paper, in particular toilet paper, for cleansing of the skin and/or mucous membrane.

The present invention further concerns a tissue paper product, in particular a toilet paper product, according to claim 19

The present description further concerns a tissue paper product, in particular a toilet paper product, comprising a composition comprising:
- at least 70 % by weight of water, relative to the total weight of the composition;
- 0.1 to 1 % by weight of at least one gelling agent, relative to the total weight of the composition, and
- 0.1 to 10 % by weight of at least one surfactant including at least one foaming surfactant, relative to the total weight of the composition.

The present description also concerns a tissue paper product, in particular a toilet paper product, comprising a composition according to the invention.

Other advantages and features of the present invention may be better understood with respect to the following examples given for illustrative purposes and the accompanying figures:
- Figures 1 and 2 represent the equipment for measuring the "soak through properties" of different compositions including the composition of the invention, i.e. the time required for a composition to soak through toilet paper without applied pressure. A detailed description of the equipment in Figures 1 and 2 is provided in the examples, i.e. Method 1;
- Figure 3 represents the equipment for measuring the stability of the foam for different compositions. A detailed description of the equipment used is provided in the examples, i.e. Method 3;
- Figure 4 represents the stability of the foam (the amount of foam transformed back into liquid over time) measured for different compositions for compositions: no. 3 and Aaah^{®} without a gelling agent (comparative examples 1 and 2), and compositions nos. 56 to 59 according to the invention containing a gelling agent (0.45% and 0.25% by weight of Carbopol Ultrez 10 respectively for compositions nos. 56 and 57; and 0.45% and 0.25% by weight of Natrosol HHX respectively for compositions nos. 58 and 59);
- Figure 5 represents the equipment used for determining the strength of the toilet paper onto which different foamed compositions are applied. A detailed description of the equipment used is provided in the examples, i.e. Method 4.

### EXAMPLES

### EXAMPLES 1 to 4: Compositions according to the invention

Compositions nos. 56 to 59 according to the invention were prepared in a conventional manner, for example, by simple admixture of components at room temperature (20°C ± 5°C), and tested.

The amount of each component of the composition is given in % by weight relative to the total weight of the composition. The pH of the different compositions is 5.7 ± 0.2. Products no. 56 to 59 are compositions according to the invention comprising a gelling agent.

**Phase A**

| **INCI name** | **Commercial name/Supplier** | **Function** | **Product no. 56** | **Product no. 57** | **Product no. 58** | **Product no. 59** |
|---|---|---|---|---|---|---|
| Water (Aqua) | Purified water/ETC | Solvent | 88.35% | 90.05% | 90.90% | 91.05% |
| Butylene Glycol | 1,3-Butanediol/ Sigma Aldrich | Emollient | 2.00% | 2.00% | 2.00% | 2.00% |
| Panthenol | D-Panthenol USP/BASF | Emollient | 0.20% | 0.20% | 0.20% | 0.20% |
| Tetrasodium EDTA | EDTA 4Na/ Sigma Aldrich | Chelating agent | 0.10% | 0.10% | 0.10% | 0.10% |
| Aloe Barbadensis Leaf Juice | Aloe Vera Gel 10X AG014P/Rahn | Active ingredient | 1.00% | 1.00% | 1.00% | 1.00% |
| Carbomer | Carbopol Ultrez 10/Noveon - Gattefossé | Gelling agent | 0.45% | 0.25% | - | - |
| Hydroxyethyl cellulose | Natrosol HHX/ Hercules / Ashland | | - | - | 0.45% | 0.25% |
| | | | 92.10% | 93.60% | 94.65% | 94.60% |

**Phase B**

| **INCI name** | **Commercial name/Supplier** | **Function** | **Product no. 56** | **Product no. 57** | **Product no. 58** | **Product no. 59** |
|---|---|---|---|---|---|---|
| Cocamidopropyl betaine | Dehyton PK 45/Cognis | Foaming surfactant | 4.00% (1.80% AM)* | 4.00% (1.80% AM)* | 4.00% (1.80% AM)* | 4.00% (1.80% AM)* |
| Perfume | Fleur d'eau MIP 1101016/Expressio ns Parfumées | Perfume | 0.20% | 0.20% | 0.20% | 0.20% |
| Caprylyl Glycol | Dermosoft Octiol/ Dr Straetmans (Lucas Meyer | Preservative | 0.10% | 0.10% | 0.10% | 0.10% |
| Phenoxyethanol | Phenoxetol/SCD-Clariant | Preservative | 0.90% | 0.90% | 0.90% | 0.90% |
| | | | 5.20% | 5.20% | 5.20% | 5.20% |

| | | | | | | |
|---|---|---|---|---|---|---|
| *: amount expressed on an active material basis: the active material is the INCI component(s) being the surfactant(s) comprised in the commercial composition | | | | | | |

**Phase C**

| **INCI name** | **Commercial name/Supplier** | **Function** | **Product no. 56** | **Product no. 57** | **Product no. 58** | **Product no. 59** |
|---|---|---|---|---|---|---|
| Sodium hydroxide | Sol. NaOH 1N/ Sigma Aldrich (sol. ETC) | pH modifier | 2.70% | 1.20% | - | - |
| Citric Acid | Sol. Acide 10%/ Sigma Aldrich (sol. ETC) | Solvent/pH modifier | - | - | 0.15% | 0.20% |
| | | | 2.700% | 1.200% | 0.150% | 0.200% |

The viscosity of each composition is measured at a temperature of 20 to 25°C, using a Brookfield LV DV I+ from BROOKFIELD ENGINEERING LABORATORIES, INC, as indicated previously. The results are summarized in the table below.

| | **Product no. 56** | **Product no. 57** | **Product no. 58** | **Product no. 59** |
|---|---|---|---|---|
| **Viscosity (Brookfield LV DV I+)** | 0.220 Pa.s (220 cP) (sp2, v50) | 0.010 Pa.s (10 cP) (sp2, v100) | 0.18 Pa.s (180cP) (sp2, v50) | 0.02 Pa.s (20 cP) (sp1, v100) |

### COMPARATIVE EXAMPLE 1: Composition without a gelling agent

Comparative composition no. 3 was prepared according to the same experimental procedure as for composition no. 58 in Examples 1 to 4.

The only difference between composition no. 3 and composition no. 58 is the absence of a gelling agent and the amount of water.

**Phase A**

| **INCI name** | **Commercial name/Supplier** | **Function** | **Product no. 03** |
|---|---|---|---|
| Water (Aqua) | Purified water/ETC | Solvent | 91.35% |
| Butylene Glycol | 1,3-Butanediol/ Sigma Aldrich | Emollient | 2.00% |
| Panthenol | D-Panthenol USP/BASF | Emollient | 0.20% |
| Tetrasodium EDTA | EDTA 4Na/ Sigma Aldrich | Chelating agent | 0.10% |
| Aloe Barbadensis Leaf Juice | Aloe Vera Gel 10X AG014P/Rahn | Active ingredient | 1.00% |
| | | | 94.65% |

**Phase B**

| **INCI name** | **Commercial name/Supplier** | **Function** | **Product no. 03** |
|---|---|---|---|
| Cocamidopropyl betaine | Dehyton PK 45/Cognis | Foaming surfactant | 4.00% (1,80% AM)* |
| Perfume | Fleur d'eau MIP 1101016/Expressions Parfumées | Perfume | 0.20% |
| Caprylyl Glycol | Dermosoft Octiol/ Dr Straetmans (Lucas Meyer | Preservative | 0.10% |
| Phenoxyethanol | Phenoxetol/SCD-Clariant | Preservative | 0.90% |
| | | | 5.20% |

| | | | |
|---|---|---|---|
| *: amount expressed on an active material basis: the active material is the INCI component(s) being the surfactant(s) comprised in the commercial composition | | | |

**Phase C**

| **INCI name** | **Commercial name/Supplier** | **Function** | **Product no. 03** |
|---|---|---|---|
| Sodium hydroxide | Sol. NaOH 1N/ Sigma Aldrich (sol. ETC) | pH modifier | - |
| Citric Acid | Sol. Acide 10%/ Sigma Aldrich (sol. ETC) | Solvent/pH modifier | 0.15% |
| | | | 0.15% |

| | | | |
|---|---|---|---|
| Composition no. 3 is a liquid. | | | |

### COMPARATIVE EXAMPLE 2: Composition Aaah^{®}, commercialized by Aaah Company

Commercial composition Aaah^{®}, sold by The Aaah Company LLC 6590 S Vine Street # 112 Centennial, CO 80121-2762 has the following composition:

| **INCI name** | **Function** |
|---|---|
| Water (Aqua) | Solvent |
| Cocamidopropyl betaine | Foaming surfactant |
| Glycerin | Emollient |
| Sodium chlorate | Deodorant |
| Whole leaf aloe vera concentrate | Natural extract |
| Commiphora Gileadenis (balm of Gilead) extract | Natural extract |
| Allantoin | Soothing agent |

Aaah^{®} composition differs from compositions nos. 56 to 59 essentially by the absence of gelling agent, pH modifier, chelating agent and perfume.

Products nos. 56 to 59 (according to the invention) were tested and compared to product no. 3 and commercialized Aaah^{®} product.

Each composition was transformed into a foam by a foaming pump dispenser, reference F2-L11 manufactured by the company Rexam, and applied on sheets of toilet paper.

Several toilet papers were tested:

### 2-ply toilet paper:

- Lotus Confort: 1-ply CWP + 1-ply TAD (100% virgin fibers); weight 38.5g/m²
- Auchan 2-ply; 2-ply CWP (100% virgin fibers); weight 38.5g/m²
- Lotus Professional Next Turn Compact 2-ply CWP (100% recycled fibers); weight 30g/m²

### 3-ply toilet paper:

### Moltonel; 3-ply CWP (100% virgin fibers); weight 54g/m²

Several methods have been used to compare the effectiveness of different foams.

### Method 1: Measure of the time required for the composition to soak through toilet paper without applied pressure

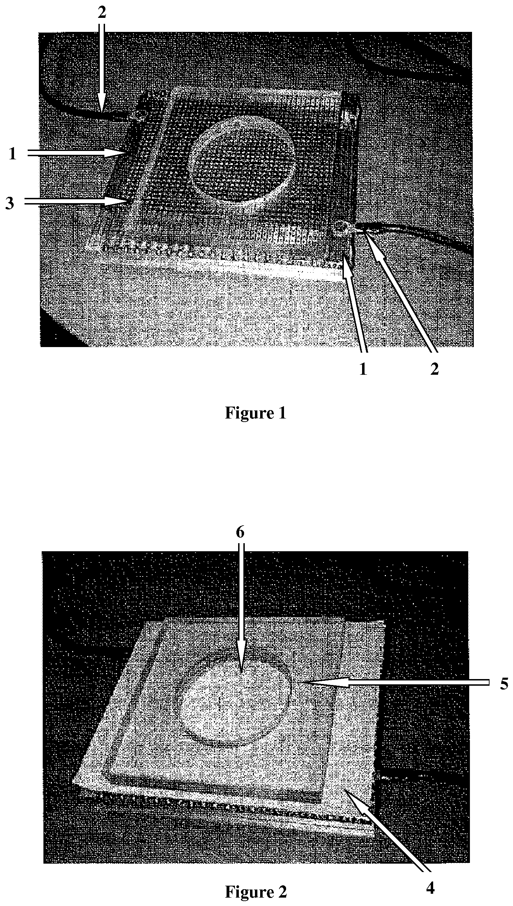

The sensor shown in Figure 1 is used to measure the time required for a liquid composition to soak through the toilet paper. The sensor consists of a printed circuit (1). This circuit has two terminals (2), each of which is connected to a series of conductive wires (3). The wires are intertwined so that each wire of a terminal is positioned between the two wires of the other terminal. All of the wires are parallel to each other, have a width of 1.8 mm and are spaced 0.7 mm. The terminals (2) are connected to a lamp (not represented). When a conductive liquid is poured onto the printed circuit (1), the electrical current is conducted by the liquid between the two terminals (2) and lights the lamp.

Several sheets (i.e. 2 or 6 sheets) of toilet paper (4) were placed on the printed circuit and maintained by a plastic plate (5) with a hole (6) so that they remain flat against the printed circuit. This is shown in Figure 2. A dose of foam is sprayed onto the top sheet at a distance of between 30 and 60 mm, with an angle of between 45 and 90 ° from the vertical. A dose of foam equals to 0.75g of the composition. At the same time a chronometer is started. As soon as the lamp (not represented) is lit, the chronometer is stopped and the time required for the fluid to pass through the two sheets (4) is noted.

The method is repeated identically for each product and each toilet paper. The following results are obtained (average of 5 measurements).

| | | | | **Time required for the liquid composition to soak through the toilet paper and trigger the sensor in seconds** | | | | | |
|---|---|---|---|---|---|---|---|---|---|
| | **No. of plies** | **No. of sheets super-posed** | **No. of doses of foam** | **Aaah^{®} Product** | **Product no.3** | **Product no. 56** | **Product no.57** | **Product no. 58** | **Product no. 59** |
| | | | | - | - | 0.45% Carbomer | 0.25% Carbomer | | |
| | | | | | | | | 0.45% Hydroxyethylcellulose | 0.25% Hydroxyethyl-cellulose |
| Lotus Confort | 2 | 2 | 1 | 1.4 | 1.3 | 132 | 21 | 28 | 4 |
| Auchan Pure Douceur | 2 | 2 | 1 | 0.8 | 0.7 | 80 | 13 | 17 | 1.7 |
| Lotus Professional | 2 | 6 | 1 | 1.0 | 1.0 | >240 | 199 | 14 | 2.3 |
| Moltonel | 3 | 2 | 1 | 2.4 | 2.4 | 319 | 85 | 79 | 5.0 |

These results show that the time required for the compositions according to the invention, which contain a gelling agent, to soak through two superposed sheets of the different toilet papers used, is more than two times greater than compositions no. 3 and Aaah^{®} which do not contain any gelling agent. The improved "soak though" property (soak through speed is reduced) of the compositions of the invention is thus due to the presence of a gelling agent.

### Method 2: Measure of the time required for the composition to soak through toilet paper with applied pressure

A pile of toilet paper is prepared by superposing several sheets (i.e. 4, 6 or 8 sheets) one on another. This pile is placed the printed circuit. A dose of foam is sprayed onto the top sheet at a distance of between 30 and 60 mm, with an angle of between 45 and 90 ° from the vertical. At the same time a chronometer is started. A plastic plate weighing 194g with a surface area of 81cm² is placed on the foam as quickly as possible. As soon as the lamp is lit, the chronometer is stopped and the time required for the fluid to soak through the four sheets is noted.
The following results are obtained (average of 5 measurements).

| | | | | **Time required for the liquid composition to soak through the toilet paper and trigger the sensor in seconds** | | | | | |
|---|---|---|---|---|---|---|---|---|---|
| | **No. of plies** | **No. of sheets super-posed** | **No. of doses of foam** | **Aaah^{®} Product** | **Product no.3** | **Product no. 56** | **Product no.57** | **Product no. 58** | **Product no. 59** |
| | | | | **-** | **-** | 0.45% Carbomer | 0.25% Carbomer | | |
| | | | | | | | | 0.45% HEC | 0.25% HEC |
| Lotus Confort | 2 | 4 | 1 | 0 | 0 | 116 | 75 | 27.2 | 4 |
| Auchan Pure Douceur | 2 | 6 | 1 | 0.6 | 0.8 | >240 | >240 | 25 | 3 |
| Lotus Professional | 2 | 8 | 1 | 0.1 | 0.5 | >240 | >240 | >240 | 5 |
| Moltonel | 3 | 4 | 1 | 0.8 | 1.6 | >240 | 53 | >240 | 49 |

From the results obtained, it appears clearly that the time required for the compositions according to the invention, which contain a gelling agent, to soak through four superposed sheets of the different toilet papers used, even when pressure is applied, is more than two times greater than compositions no. 3 and Aaah^{®} which do not contain any gelling agent. These results show the improved "soak through" property (soak through speed is reduced) of the compositions of the invention due to the presence of the gelling agent.

### Method 3: Measure of foam stability

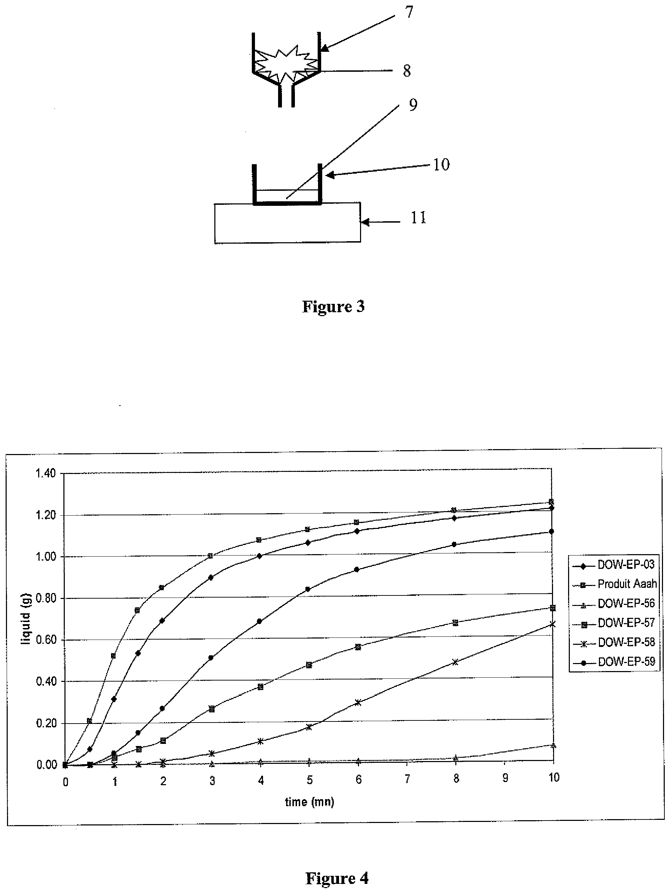

The amount of foam transformed back into liquid over time shows the stability of a composition. The stability of compositions nos. 56 to 59 (according to the invention) was measured and compared to the stability of compositions no. 3 and Aaah^{®}, using the equipment illustrated in Figure 3. Two doses of foam (8) are sprayed in the funnel (7), and at the same time a chronometer is started. The weight of liquid (9) collected in recipient (10) and displayed on the scale (11) is regularly noted. The results are illustrated in Figure 4.

Based on the results illustrated in Figure 4, it appears that the compositions according to the present invention are transformed into liquid at least two times less faster that the comparative compositions (no. 3 and Aaah^{®}) which do not contain any gelling agent.

Based on these results, it is clear that the presence of a gelling agent improves the stability of the compositions.

### Method 4: Measure of the time required for the tissue paper onto which the foam is applied to tear under a mechanical stress (strength of the paper)

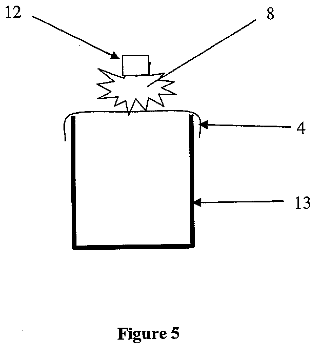

Several sheets (i.e. 2 or 3 sheets) of toilet paper (4) are fixed on a 53 mm diameter cylindrical container (13) with a rubber band (not represented) so as to have an evenly distributed voltage. A dose of foam (8) is sprayed onto the top sheet at a distance of between 30 and 60 mm, with an angle of between 45 and 90° from the vertical. A cylindrical weight (12) having a diameter of 20 mm, a height of 21.5 mm and a weight of 50 g, is placed on the sheet. This is illustrated in Figure 5. At the same time a chronometer is started. When the sheet (4) is torn by the action of weight (12) and liquid, the chronometer is stopped and the time required by the liquid to tear the several sheets is noted.
The following results are obtained (average of 5 measurements).

| | | | | **Time required for the liquid composition to soak through the toilet paper and trigger the sensor in seconds** | | | | |
|---|---|---|---|---|---|---|---|---|
| | **No. of plies** | **No. of sheets super-posed** | **No. of doses of foam** | **Aaah^{®} Product** | **Product no.3** | **Product no. 56** | **Product no.57** | **Product no. 58** |
| | | | | - | - | 0.45% Carbomer | 0.25% Carbomer | |
| | | | | | | | | 0.45% HEC |
| Lotus Confort | 2 | 3 | 1 | 8 | 4 | >400 | >400 | 44 |
| Auchan Pure Douceur | 2 | 3 | 1 | 1 | 1 | 50 | 10 | 16 |
| Lotus Professional | 2 | 3 | 1 | 1 | 1 | 153 | 19 | 3 |
| Moltonel | 3 | 2 | 1 | 10 | 11 | 119 | 43 | 15 |

It appears from these results that under mechanical stress, the time required for superposed sheets of the toilet paper onto which the foam is applied, to tear, is more than two times less than the time required for superposed sheets of the toilet paper onto which comparative compositions (no. 3 and Aaah^{®}) without a gelling agent are applied, to tear.

### EXAMPLES 5 and 6: Compositions according to the invention

Compositions nos. 55 and 60 according to the invention were prepared in a conventional manner, for example, by simple admixture of components at room temperature (20°C ± 5°C), and tested.

The amount of each component of the composition is given in % by weight relative to the total weight of the composition. The pH of the different compositions is 5.7 ± 0.2.

**Phase A**

| **INCI name** | **Commercial name/Supplier** | **Function** | **Product no. 55** | **Product no. 60** |
|---|---|---|---|---|
| Water (Aqua) | Purified water/ETC | Solvent | 88.30% | 90.10% |
| Butylene Glycol | 1,3-Butanediol/ Sigma Aldrich | Emollient | 2.00% | 2.00% |
| Panthenol | D-Panthenol USP/BASF | Emollient | 0.20% | 0.20% |
| Tetrasodium EDTA | EDTA 4Na/ Sigma Aldrich | Chelating agent | 0.10% | 0.10% |
| Aloe Barbadensis Leaf Juice | Aloe Vera Gel 10X AG014P/Rahn | Active ingredient | 1.00% | 1.00% |
| Carbomer | Carbopol Ultrez 10/ Noveon - Gattefossé | Gelling agent | 0.5% | 0.20% |
| | | | 92.10% | 93.60% |

**Phase B**

| **INCI name** | **Commercial name/Supplier** | **Function** | **Product no. 55** | **Product no. 60** |
|---|---|---|---|---|
| Cocamid o-propyl betaine | Dehyton PK 45/Cognis | Foaming surfactant | 4.00% (1,80%AM)* | 4.00% |
| Perfume | Fleur d'eau MIP 1101016/Expression s Parfumées | Perfume | 0.20% | 0.20% |
| Caprylyl Glycol | Dermosoft Octiol/ Dr Straetmans (Lucas Meyer | Preservative | 0.10% | 0.10% |
| Phenoxy -ethanol | Phenoxetol/SCD-Clariant | Preservative | 0.90% | 0.90% |
| | | | 5.20% | 5.20% |

| | | | | |
|---|---|---|---|---|
| *: amount expressed on an active material basis: the active material is the INCI component(s) being the surfactant(s) comprised in the commercial composition | | | | |

**Phase C**

| **INCI name** | **Commercial name/Supplier** | **Function** | **Product no. 55** | **Product no. 60** |
|---|---|---|---|---|
| Sodium hydroxide | Sol. NaOH 1N/ Sigma Aldrich (sol. ETC) | pH modifier | 2.70% | 1.20% |
| | | | 2.700% | 1.200% |

The viscosity of each composition is measured at a temperature of 25°C, using a Brookfield LV DV I + from BROOKFIELD ENGINEERING LABORATORIES, INC, as indicated previously.

Composition no. 55 showed a higher viscosity than composition no. 56, which may render the pumping slightly more difficult. The stability of this composition is lower than that of composition no. 56 but higher than the stability of comparative examples 1 and 2.

Composition no. 60 showed a lower viscosity than composition no. 57. Its soak through properties are not as good as composition no. 57 but still remain satisfactory.

## Claims

1. A foamable composition comprising:
- at least 85% by weight of water, relative to the total weight of the composition;
- 0.25 to 0.45% by weight of at least one gelling agent, relative to the total weight of the composition, and
- 0.1 to 10 % by weight of at least one surfactant including at least one foaming surfactant, relative to the total weight of the composition, wherein
the gelling agent is chosen from carbomers, hydroxyethylcellulose (HEC), and mixtures thereof;
the foaming surfactant is a betaine derivative chosen from alkylbetaines such as cocobetaine, alkylamidobetaines such as cocamidopropylbetaine, and mixtures thereof.

2. The composition according to claim 1, wherein said composition satisfies at least one of the following conditions:
- once transformed into foam and applied onto the surface of several sheets of tissue paper placed one on top of each other, the time required for said composition to soak through said sheets of paper, with or without applied pressure, is at least two times greater than the time required for a composition without a gelling agent to soak through said sheets of paper, and/or
- the foam stability of said composition is such that after two minutes, the said composition is transformed into liquid at least two times less faster than a composition without a gelling agent, and/or
- once transformed into foam and applied onto the surface of several sheets of tissue paper, the time required to tear said sheets of paper under a mechanical stress, is at least two times less than the time required to tear said sheets of paper onto which a composition without a gelling agent is applied.

3. The composition according to claim 1 or 2, wherein once transformed into foam and applied onto the surface of 2 sheets of tissue paper placed one on top of each other, the time required for said composition to soak through said sheets of paper without applied pressure, is at least two times greater than the time required for a composition without a gelling agent to soak through said sheets of paper.

4. The composition according to any one of claims 1 to 3, wherein once transformed into foam and applied onto the surface of 4 sheets of tissue paper placed one on top of each other, the time required for said composition to soak through said sheets of paper with applied pressure, is at least two times greater than the time required for a composition without a gelling agent to soak through said sheets of paper.

5. The composition according to any one of claims 1 to 4, wherein once transformed into foam and applied onto the surface of 3 sheets of tissue paper placed one on top of each other, the time required to tear said sheets of paper under a mechanical stress, is at least two times less than the time required to tear said sheets of paper onto which a composition without a gelling agent is applied.

6. The composition according to any one of claims 2 to 5, wherein the tissue paper is a 1-ply CWP + 1-ply TAD toilet paper weighing 38.5 g/m²

7. The composition according to any one of claims 1 to 6, wherein said composition has a viscosity ranging from 0.001 to 0.500 Pa.s.

8. The composition according to any one of claims 1 to 7, wherein the amount of water ranges from 85% to 95% by weight, relative to the total weight of the composition.

9. The composition according to any one of claims 1 to 8, wherein the total amount of foaming surfactant(s) ranges from 0.5 to 6 % by weight, relative to the total weight of the composition.

10. The composition according to any one of claims 1 to 9, wherein the composition further contains one or more surfactants chosen from anionic surfactants, cationic surfactants, amphoteric surfactants, and mixtures thereof.

11. The composition according to any one of claims 1 to 10, wherein the total amount of surfactant(s) ranges from 0.5 to 6 % by weight relative to the total weight of the composition.

12. The composition according to any one of claims 1 to 11, containing further additives and/or active agents chosen from preservatives or preserving agents; sequestering or chelating agents; antioxidants; fragrances; pH modifiers; antiseptics; antimicrobials; natural extracts having soothing, moisturizing, and healing properties; soothing agents or emollients; and mixtures thereof.

13. Use of a composition according to any one of claims 1 to 12 for cleansing of the skin and/or mucous membrane.

14. Use of a composition according to any one of claims 1 to 12 with tissue paper, in particular toilet paper, for cleansing of the skin and/or mucous membrane.

15. A kit for cleansing of the skin and/or mucous membranes comprising the composition according to any one of claims 1 to 12 and a dispensing foam pump container.

16. The kit according to claim 15 wherein said kit further comprises tissue paper, in particular toilet paper.

17. A container provided with a dispensing foam pump and containing a composition according to any one of claims 1 to 12.

18. An automatic foam dispenser containing a composition according to any one of claims 1 to 12.

19. A tissue paper product, in particular a toilet paper product, comprising a foamable composition comprising:
- at least 85% by weight of water, relative to the total weight of the composition;
- 0.25 to 0.45% % by weight of at least one gelling agent, relative to the total weight of the composition, and
- 0.1 to 10% by weight of at least one surfactant including at least one foaming surfactant, relative to the total weight of the composition, wherein
the gelling agent is chosen from carbomers, hydroxyethylcellulose (HEC), and mixtures thereof;
the foaming surfactant is a betaine derivative chosen from alkylbetaines such as cocobetaine, alkylamidobetaines such as cocamidopropylbetaine, and mixtures thereof.

## Patentansprüche

1. Schäumbare Zusammensetzung, umfassend:
- mindestens 85 Gew.% Wasser mit Bezug auf das Gesamtgewicht der Zusammensetzung;
- 0,25 bis 0,45 Gew.% mindestens eines Geliermittels mit Bezug auf das Gesamtgewicht der Zusammensetzung, und
- 0,1 bis 10 Gew.% mindestens eines oberflächenaktiven Mittels, darin eingeschlossen mindestens ein schäumbares oberflächenaktives Mittel mit Bezug auf das Gesamtgewicht der Zusammensetzung, wobei
das Geliermittel ausgewählt ist aus Carbomeren, Hydroxyethylcellulose (HEC) und Mischungen daraus;
das schäumbare oberflächenaktive Mittel ein Betain-Derivat ist, ausgewählt aus Alkylbetainen wie z. B. Cocobetain, Alkylamidobetainen, wie z. B. Cocamidopropylbetain und Mischungen daraus.

2. Zusammensetzung nach Anspruch 1, wobei die Zusammensetzung mindestens einer der folgenden Bedingungen genügt:
- nachdem sie in Schaum umgewandelt und auf die Oberfläche von mehreren Bahnen von Tissuepapier, die aufeinander angeordnet sind, aufgetragen ist, ist die Zeit, die erforderlich ist, damit die Zusammensetzung durch die Bahnen von Papier mit oder ohne angewendeten Druck dringt, mindestens zweimal länger als die Zeit, die für eine Zusammensetzung ohne ein Geliermittel erforderlich ist, damit sie durch die Bahnen von Papier dringt, und/oder
- die Schaumstabilität der Zusammensetzung derart ist, dass nach zwei Minuten die Zusammensetzung mindestens zweimal langsamer als eine Zusammensetzung ohne ein Geliermittel in Flüssigkeit umgewandelt wird, und/oder
- nachdem sie in Schaum umgewandelt und auf die Oberfläche von mehreren Bahnen von Tissuepapier aufgetragen ist, die Zeit, die erforderlich ist, um die Bahnen von Papier unter einer mechanischen Spannung zu zerreißen, mindestens zweimal kürzer ist als die Zeit, die erforderlich ist, um die Bahnen von Papier, auf die eine Zusammensetzung ohne ein Geliermittel angewendet wurde, zu zerreißen.

3. Zusammensetzung nach Anspruch 1 oder 2, wobei, nachdem sie in Schaum umgewandelt und auf die Oberfläche von 2 Bahnen von Tissuepapier, die aufeinander angeordnet sind, aufgetragen ist, die Zeit, die erforderlich ist, damit die Zusammensetzung durch die Bahnen von Papier ohne angewendeten Druck dringt, mindestens zweimal länger als die Zeit ist, die für eine Zusammensetzung ohne ein Geliermittel erforderlich ist, damit sie durch die Bahnen von Papier dringt.

4. Zusammensetzung nach einem der Ansprüche 1 bis 3, wobei, nachdem sie in Schaum umgewandelt und auf die Oberfläche von 4 Bahnen von Tissuepapier, die aufeinander angeordnet sind, aufgetragen ist, die Zeit, die erforderlich ist, damit die Zusammensetzung durch die Bahnen von Papier mit angewendeten Druck dringt, mindestens zweimal länger als die Zeit ist, die für eine Zusammensetzung ohne ein Geliermittel erforderlich ist, damit sie durch die Bahnen von Papier dringt.

5. Zusammensetzung nach einem der Ansprüche 1 bis 4, wobei, nachdem sie in Schaum umgewandelt und auf die Oberfläche von 3 Bahnen von Tissuepapier, die aufeinander angeordnet sind, aufgetragen ist, die Zeit, die erforderlich ist, um die Bahnen von Papier unter einer mechanischen Spannung zu zerreißen, mindestens zweimal kürzer ist als die Zeit, die erforderlich ist, um die Bahnen von Papier, auf die eine Zusammensetzung ohne ein Geliermittel angewendet wurde, zu zerreißen.

6. Zusammensetzung nach einem der Ansprüche 2 bis 5, wobei das Tissuepapier ein 1-lagiges CWP- + 1-lagiges TAD-Toilettenpapier mit einem Gewicht von 38,5 g/m² ist.

7. Zusammensetzung nach einem der Ansprüche 1 bis 6, wobei die Zusammensetzung eine Viskosität im Bereich von 0,001 bis 0,500 Pa.s. aufweist.

8. Zusammensetzung nach einem der Ansprüche 1 bis 7, wobei die Menge von Wasser im Bereich von 85 Gew.% bis 95 Gew.% mit Bezug auf das Gesamtgewicht der Zusammensetzung liegt.

9. Zusammensetzung nach einem der Ansprüche 1 bis 8, wobei die Gesamtmenge von schäumbaren(em) oberflächenaktiven(em) Mittel(n) im Bereich von 0,5 bis 6 Gew.% mit Bezug auf das Gesamtgewicht der Zusammensetzung liegt.

10. Zusammensetzung nach einem der Ansprüche 1 bis 9, wobei die Zusammensetzung weiter ein oder mehrere oberflächenaktive Mittel umfasst, ausgewählt aus anionischen oberflächenaktiven Mitteln, kationischen oberflächenaktiven Mitteln, amphoteren oberflächenaktiven Mitteln und Mischungen daraus.

11. Zusammensetzung nach einem der Ansprüche 1 bis 10, wobei die Gesamtmenge von oberflächenaktiven(em) Mittel(n) im Bereich von 0,5 bis 6 Gew.% mit Bezug auf das Gesamtgewicht der Zusammensetzung liegt.

12. Zusammensetzung nach einem der Ansprüche 1 bis 11, weiter umfassend Zusatzstoffe und/oder Wirkstoffe, ausgewählt aus Konservierungsmitteln oder konservierenden Mitteln; Markierungsmitteln oder Chelatbildnern; Antioxidationsmitteln; Riechstoffen; pH-Modifikationsmitteln; antiseptischen Mitteln; antimikrobiellen Mitteln; natürlichen Extrakten mit beruhigenden, befeuchtenden und heilenden Eigenschaften; Beruhigungsmitteln oder Weichmachern; und Mischungen daraus.

13. Verwendung einer Zusammensetzung nach einem der Ansprüche 1 bis 12 zur Reinigung der Haut und/oder der Schleimhaut.

14. Verwendung einer Zusammensetzung nach einem der Ansprüche 1 bis 12 mit Tissuepapier, insbesondere Toilettenpapier, zur Reinigung der Haut und/oder der Schleimhaut.

15. Kit zur Reinigung der Haut und/oder der Schleimhäute, umfassend die Zusammensetzung nach einem der Ansprüche 1 bis 12 und einen Abgabeschaumpumpenbehälter.

16. Kit nach Anspruch 15, wobei das Kit weiter Tissuepapier, insbesondere Toilettenpapier, umfasst.

17. Behälter, ausgestattet mit einer Abgabeschaumpumpe und enthaltend eine Zusammensetzung nach einem der Ansprüche 1 bis 12.

18. Automatische Schaumabgabevorrichtung, enthaltend eine Zusammensetzung nach einem der Ansprüche 1 bis 12.

19. Tissuepapierprodukt, insbesondere Toilettenpapierprodukt, umfassend eine schäumbare Zusammensetzung, umfassend:
- mindestens 85 Gew.% Wasser mit Bezug auf das Gesamtgewicht der Zusammensetzung;
- 0,25 bis 0,45 Gew.% mindestens eines Geliermittels mit Bezug auf das Gesamtgewicht der Zusammensetzung; und
- 0,1 bis 10 Gew.% mindestens eines oberflächenaktiven Mittels, darin eingeschlossen mindestens ein schäumbares oberflächenaktives Mittel mit Bezug auf das Gesamtgewicht der Zusammensetzung, wobei
das Geliermittel ausgewählt ist aus Carbomeren, Hydroxyethylcellulose (HEC) und Mischungen daraus;
das schäumbare oberflächenaktive Mittel ein Betain-Derivat ist, ausgewählt aus Alkylbetainen wie z. B. Cocobetain, Alkylamidobetainen wie z. B. Cocamidopropylbetain und Mischungen daraus.

## Revendications

1. Composition moussable comprenant :
- au moins 85 % en poids d'eau, par rapport au poids total de la composition ;
- 0,25 à 0,45 % en poids d'au moins un agent gélifiant, par rapport au poids total de la composition, et
- 0,1 à 10 % en poids d'au moins un tensioactif incluant au moins un tensioactif moussant, par rapport au poids total de la composition, dans laquelle
l'agent gélifiant est choisi parmi des carbomères, l'hydroxyéthylcellulose (HEC), et leurs mélanges ;
le tensioactif moussant est un dérivé de bétaïne choisi parmi les alkylbétaïnes telles que la cocobétaïne, les alkylamidobétaïnes telles que la cocamidopropylbétaïne, et leurs mélanges.

2. Composition selon la revendication 1, dans laquelle ladite composition satisfait au moins l'une des conditions suivantes :
- une fois transformée en mousse et appliquée sur la surface de plusieurs feuilles de papier tissue placées au-dessus les unes des autres, le temps requis pour que ladite composition traverse lesdites feuilles de papier tissue, avec ou sans pression appliquée, est au moins deux fois plus grand que le temps requis pour qu'une composition sans agent gélifiant traverse lesdites feuilles de papier tissue, et/ou
- la stabilité de mousse de ladite composition est telle qu'après deux minutes, ladite composition est transformée en liquide au moins deux fois moins vite qu'une composition sans agent gélifiant, et/ou
- une fois transformée en mousse et appliquée sur la surface de plusieurs feuilles de papier tissue, le temps requis pour déchirer lesdites feuilles de papier sous une contrainte mécanique, est au moins deux fois moins que le temps requis pour déchirer lesdites feuilles de papier tissue sur lesquelles une composition sans agent gélifiant est appliquée.

3. Composition selon la revendication 1 ou 2, dans laquelle une fois transformée en mousse et appliquée sur la surface de 2 feuilles de papier tissue placées au-dessus les unes des autres, le temps requis pour que ladite composition traverse lesdites feuilles de papier tissue sans pression appliquée, est au moins deux fois plus grand que le temps requis pour qu'une composition sans agent gélifiant traverse lesdites feuilles de papier tissue.

4. Composition selon l'une quelconque des revendications 1 à 3, dans laquelle une fois transformée en mousse et appliquée sur la surface de 4 feuilles de papier tissue placées au-dessus les unes des autres, le temps requis pour que ladite composition traverse lesdites feuilles de papier tissue avec une pression appliquée, est au moins deux fois plus grand que le temps requis pour qu'une composition sans agent gélifiant traverse lesdites feuilles de papier tissue.

5. Composition selon l'une quelconque des revendications 1 à 4, dans laquelle une fois transformée en mousse et appliquée sur la surface de 3 feuilles de papier tissue placées au-dessus les unes des autres, le temps requis pour déchirer lesdites feuilles de papier tissue sous une contrainte mécanique est au moins deux fois moins que le temps requis pour déchirer lesdites feuilles de papier tissue sur lesquelles une composition sans agent gélifiant est appliquée.

6. Composition selon l'une quelconque des revendications 2 à 5, dans laquelle le papier tissue est un papier toilette CWP 1 pli + TAD 1 pli pesant 38,5 g/m².

7. Composition selon l'une quelconque des revendications 1 à 6, dans laquelle ladite composition a une viscosité variant de 0,001 à 0,500 Pa.s.

8. Composition selon l'une quelconque des revendications 1 à 7, dans laquelle la quantité d'eau varie de 85 % à 95 % en poids, par rapport au poids total de la composition.

9. Composition selon l'une quelconque des revendications 1 à 8, dans laquelle la quantité totale de tensioactif (s) moussant (s) varie de 0,5 à 6 % en poids, par rapport au poids total de la composition.

10. Composition selon l'une quelconque des revendications 1 à 9, dans laquelle la composition contient en outre un ou plusieurs tensioactifs choisis parmi les tensioactifs anioniques, les tensioactifs cationiques, les tensioactifs amphotères, et leurs mélanges.

11. Composition selon l'une quelconque des revendications 1 à 10, dans laquelle la quantité totale de tensioactif (s) varie de 0,5 à 6 % en poids, par rapport au poids total de la composition.

12. Composition selon l'une quelconque des revendications 1 à 11, contenant en outre des additifs et/ou des agents actifs choisis parmi les conservateurs ou les agents de conservation ; les agents séquestrants ou chélatants ; les antioxydants ; les parfums ; les modificateurs de pH ; les antiseptiques ; les antimicrobiens ; les extraits naturels ayant des propriétés calmantes, hydratantes et cicatrisantes ; les agents calmants ou les émollients ; et leurs mélanges.

13. Utilisation d'une composition selon l'une quelconque des revendications 1 à 12, pour le nettoyage de la peau et/ou d'une membrane muqueuse.

14. Utilisation d'une composition selon l'une quelconque des revendications 1 à 12 avec du papier tissue, en particulier du papier toilette, pour le nettoyage de la peau et/ou d'une membrane muqueuse.

15. Kit de nettoyage de la peau et/ou de membranes muqueuses comprenant la composition selon l'une quelconque des revendications 1 à 12 et un contenant à pompe distributeur de mousse.

16. Kit selon la revendication 15, dans lequel ledit kit comprend en outre du papier tissue, en particulier du papier toilette.

17. Récipient pourvu d'une pompe distributeur de mousse et contenant une composition selon l'une quelconque des revendications 1 à 12.

18. Distributeur de mousse automatique contenant une composition selon l'une quelconque des revendications 1 à 12.

19. Produit de papier tissue, en particulier produit de papier toilette, comprenant une composition moussable comprenant :
- au moins 85 % en poids d'eau, par rapport au poids total de la composition ;
- 0,25 à 0,45 % en poids d'au moins un agent gélifiant, par rapport au poids total de la composition, et
- 0,1 à 10 % en poids d'au moins un tensioactif incluant au moins un tensioactif moussant, par rapport au poids total de la composition, dans lequel
l'agent gélifiant est choisi parmi des carbomères, l'hydroxyéthylcellulose (HEC), et leurs mélanges ;
le tensioactif moussant est un dérivé de bétaïne choisi parmi les alkylbétaïnes telles que la cocobétaïne, les alkylamidobétaines telles que la cocamidopropylbétaïne, et leurs mélanges.
